(19) Europäisches Patentamt
European Patent Office
Office européen des brevets

(11) Publication number: **0 277 561 B1**

(12) # EUROPEAN PATENT SPECIFICATION

(45) Date of publication of patent specification: **15.04.92**

(51) Int. Cl.5: **C07C 279/04**, C07C 279/18, C07D 249/18, C07K 1/06

(21) Application number: **88100919.5**

(22) Date of filing: **22.01.88**

(54) **New arginine derivative, process for the preparation thereof and its use in peptide synthesis.**

(30) Priority: **03.02.87 IT 1923487**

(43) Date of publication of application:
**10.08.88 Bulletin 88/32**

(45) Publication of the grant of the patent:
**15.04.92 Bulletin 92/16**

(84) Designated Contracting States:
**AT BE CH DE ES FR GB GR IT LI LU NL SE**

(56) References cited:
**EP-A- 0 137 742**
**DE-A- 2 113 707**
**GB-A- 791 319**
**US-A- 4 108 846**
**US-A- 4 477 428**
**US-A- 4 499 068**

(73) Proprietor: **ENIRICERCHE S.p.A.**
**Corso Venezia 16**
**I-20121 Milan(IT)**

Proprietor: **SCLAVO S.p.A.**
**Via Fiorentina 1**
**I-53100 Siena(IT)**

(72) Inventor: **Caciagli, Valerio**
**Via Montaione, 41**
**Roma(IT)**
Inventor: **Verdini, Antonio Silvio**
**Via S. Martino, 12**
**Monterotondo Roma(IT)**

(74) Representative: **Gervasi, Gemma, Dr. et al**
**Studio Brevetti e Marchi NOTARBARTOLO & GERVASI 33, Viale Bianca Maria**
**I-20122 Milano(IT)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention refers to a new di-protected arginine derivative, i.e. $N^{\alpha}$-fluorenylmethoxycarbonyl-$N^{G}$-trityl-arginine, a method for preparing it and its use in both free solution and solid-phase synthesis of peptides containing one or more arginine residues.

Both in classical peptide synthesis, i.e. synthesis in homogeneous solution, and in the recently developed solid-phase synthesis, side-chain protection of arginine presents several and different problems which have not yet been solved satisfactorily.

More particularly, these problems concern both the preparation of the suitably selected arginine derivative to be employed in the peptide synthesis (preparation which often proceeds with very low yields), and its use in peptide synthesis, i.e. the activation of said protected arginine, its addition to the peptide chain (steps that often lead to results different from the desired ones), and, also, its removal at the completion of the synthesis (as often removal of arginine protecting groups requires cleavage conditions severe enough to affect peptide chain integrity), mainly when the end peptide is highly heterogeneous in amino acid composition.

Among the protected arginine derivatives known in literature, those actually employed in the synthesis of peptides containing said amino acid (see for example Chem. Pharm. Bull. 29, 2825 (1981) and J.C.S. Perkin Trans. 1, 2065 (1985)) are:

a) $N^{\alpha}$-fluorenylmethoxycarbonyl-$N^{G}$-bisadamantyloxycarbonylarginine (more easily indicated, using the internationally recognized abbreviations, as Fmoc-Arg(Adoc)$_2$-OH), which is difficult to prepare and is unstable easily converting, during the synthesis, into ornithine-containing side-products;

b) $N^{\alpha}$-fluorenylmethoxycarbonyl-$N^{G}$-(4-methoxybenzenesulphonyl)-arginine (Fmoc-Arg(Mbs)-OH), which requires very severe deprotection conditions (methanesulphonic acid) that might alter, at least partially, the peptide structure, mainly if it contains Asp and Asn moieties, with formation of succinimide groups;

c) $N^{\alpha}$-t-butoxycarbonyl-$N^{G}$-(4-toluenesulphonyl)arginine (Boc-Arg(Tos)-OH), and $N^{\alpha}$-t-amyloxycarbonyl-$N^{G}$-(4-toluenesulphonyl)arginine (Aoc-Arg(Tos)-OH), which are now finding increasing application owing to the stability of the toluene-p-sulphonyl group to the conditions usually employed for the removal of t-butoxy- and t-amyloxy-carbonyl groups and to the ease of removal of said protecting group by the hydrofluoric acid procedure. These two derivatives, however, when used in carbodi-imide-mediated acylation reactions, give rise to several side-products all containing more than one arginine residue;

d) $N^{\alpha}$-fluorenylmethoxycarbonyl-arginine hydrochloride (Fmoc-Arg(HCl)-OH) which, during Fmoc cleavage by the conventional method (20 % piperidine in dimethylformamide), undergoes substantial deprotonation thus making the acylation of guanidino nitrogens, in the subsequent acylation step, possible;

e) $N^{\alpha}$-t-butoxycarbonyl-$N^{G}$-(4-methoxy-2,3,6-trimethylbenzenesulphonyl)arginine (Boc-Arg(Mtr)-OH), which actually finds wide application owing to the ease of removal of the 4-methoxy-2,3,6-trimethylbenzenesulphonyl group in very mild conditions (trifluoroacetic acid/thioanisole for about one hour); this compound however when used in dicyclohexylcarbodiimide-mediated coupling reactions, gives rise very easily to side-products containing more than one arginine residue; and, finally

f) $N^{\alpha}$-fluorenylmethoxycarbonyl-$N^{G}$-(4-methoxy-2,3,6-trimethylbenzenesulphonyl)-arginine(Fmoc-Arg(Mtr)-OH), which is widely employed in solid-phase synthesis on polyacrylamide supports. Its use however has the drawback that, in order to afford a crude reaction product in acceptable yields and reasonable purity, a quite long (about 6 hours) cleavage treatment with trifluoroacetic acid in the presence of methylethylsulfide and phenol as scavengers, is necessary.

There is need therefore for an arginine derivative with the $\alpha$-amino and guanidino functionalities protected with completely independent protecting groups which are, particularly the side-chain protecting group, easily removable in very mild conditions.

Said need is particularly felt when the solid-phase method on polyacrylamide supports, developed by Sheppard in the last few years, is employed. Said method, which has now found very wide application, owes its success not only to the idea of completely independent protecting groups of the $\alpha$-amino and the side-chain functionalities (base-labile Fmoc groups for $\alpha$-$NH_2$ and acid-labile tert-butyl-like groups for the side-chain functionalities), but also to the possibility, usually afforded by a suitably selected protecting groups combination, of cleavage from the polymer support with simultaneous removal of the acid-labile side-chain protecting groups under very mild acid conditions. When, however, the end peptide contains one or more arginine residues, the reaction conditions for the final deprotection must, for the time being, be more severe because the guanidino protecting groups employed in the synthesis are only partially removable by mild acidolysis.

It has now been found, and represents a first object of the present invention, that it is possible to solve

completely this problem by using a new arginine derivative of formula (I)

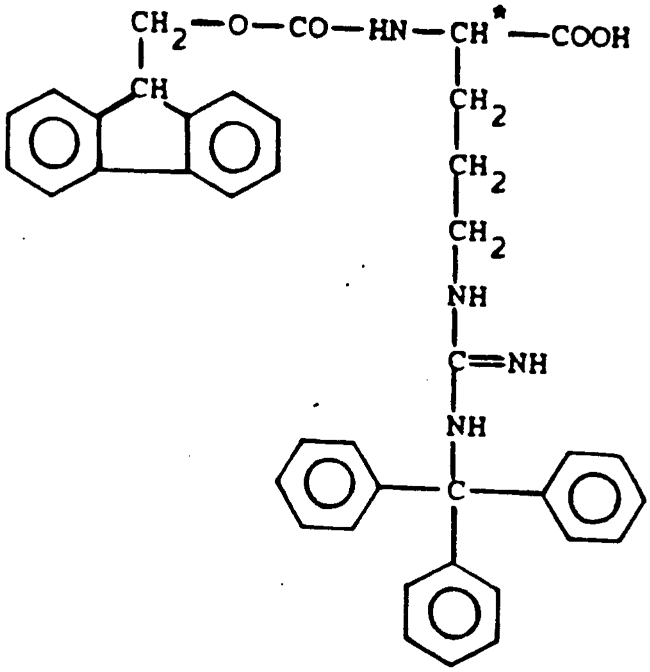

(I)

wherein the $\alpha$-amino and guanidino functionalities are protected with a fluorenylmethoxycarbonyl and a trityl group, respectively.

In formula (I) above, the asterisk means that the starred carbon atom may have either the R or the S configuration, or that the compound may be a mixture of the two isomeric forms in any proportion.

While, on the one hand it has been confirmed that the reaction conditions suitable to cleave the base-labile fluorenylmethoxycarbonyl group, do not affect the trityl protecting group, on the other hand it has been demonstrated also that cleavage of the trityl group can be obtained quickly and under very mild acid conditions, so that no alteration of the peptide structure does occur even in the presence of particularly labile amino acid residues.

More particularly it has been shown that complete removal of the trityl group at the end of the peptide synthesis, can be achieved easily and quickly (typically in 15-25 minutes), by treatment with trifluoroacetic acid in the presence of suitable scavengers capable of effectively trap the trityl radicals which form.

Particularly preferred reaction conditions include for instance the use of ethanedithiol/trifluoroacetic acid or water/trifluoroacetic acid in a 2/8 to 0.5/9.5 ratio, optionally containing additional scavengers. These conditions are in fact those used in solid-phase synthesis on polyacrylamide supports (Sheppard's method) to afford detachment of the peptide from the resin with simultaneous cleavage of the conventional acid-labile protecting groups of the side-chain functionalities.

Scavengers which can suitably be employed in the final de-protection steps are conventional thiol scavengers. According to a preferred embodiment of the present invention, in solid phase synthesis on poly-acrylamide supports, final de-protection is carried out using ethanedithiol and trifluoroacetic acid containing S-acetamidomethyl-cysteine (H-Cys(Acm)-OH).

The protected arginine derivative of the present invention can be easily coupled to the free amino-terminus of the peptide chain which is assembled attached to the resin, by activating it with dicylohexylcarbodiimide (DCCI) and N-hydroxybenzotriazole (HOBt). Unlike other protected arginine derivatives, the arginine derivative of formula (I) does not give rise to any undesired side-reaction. This achievement is of remarkable interest as it is known that with many other arginine derivatives now employed in peptide synthesis, besides lactam formation, side-reactions leading to peptides containing more than one arginine moiety in the chain, may occur with great ease.

The arginine derivative of formula (I) can be readily prepared according to the three step process, summarized in the following Scheme, which represents a second specific object of the present invention.

Scheme

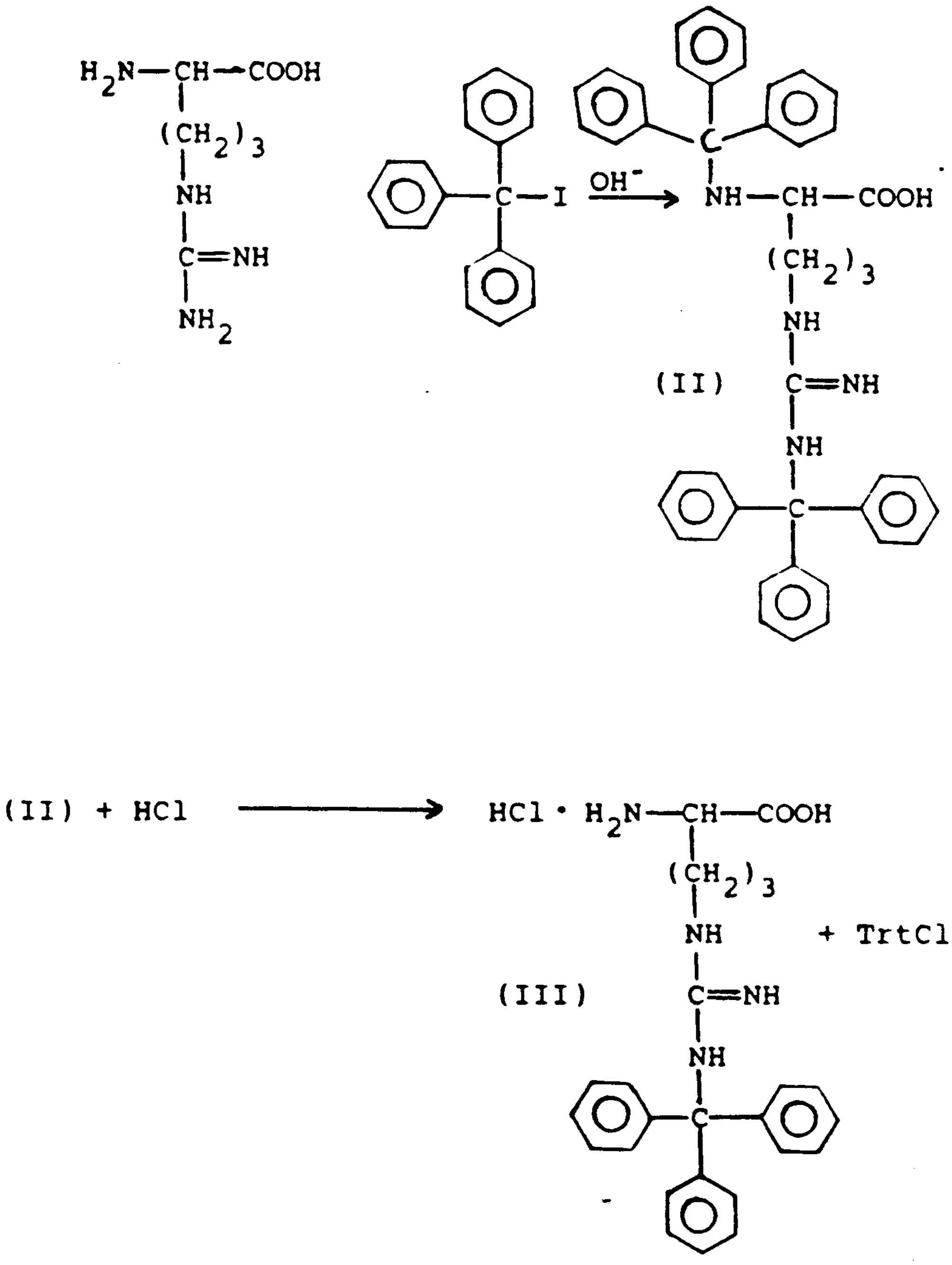

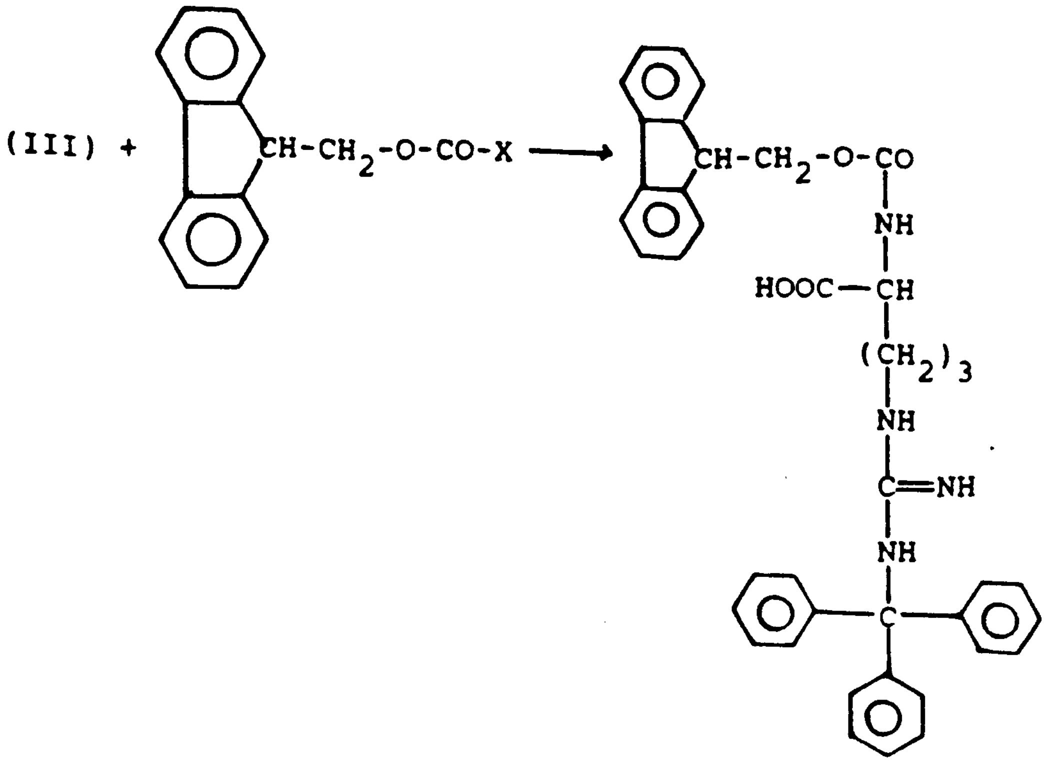

In the first step reported above, arginine is reacted with trityl iodide in the presence of an acceptor of the hydriodic acid which thus forms. As the desired product is the di-trityl derivative (II), obviously a molar amount of trityl iodide at least double than that of the starting arginine has to be employed. It is however preferable to use excess trityl iodide, typically a 50-100 % molar excess, over the stoichiometric amount. Trityl iodide may be directly contacted with arginine in the presence of the hydriodic acid acceptor or it may be formed in situ by adding trityl chloride, a commercial product, to a mixture of arginine, acid acceptor agent, and alkaline iodide.

Any inorganic base, such as an alkali metal or alkaline-earth metal hydroxide and, preferably, sodium or potassium hydroxide, may conveniently be employed as the acid acceptor agent, in this first step. The reaction may be carried out at a temperature generally comprised between room temperature and 100 °C. However, in order to avoid any possible racemization at the chiral carbon atom, while obtaining industrially acceptable yields, the reaction is preferably carried out at a temperature comprised between 30 and 50 °C. When said preferred temperature range is employed, the di-trityl derivative readily forms within one hour (typically from 15 to 45 minutes).

This first step is carried out in the presence of a solvent medium which consists of one or more polar organic solvents. Suitable solvents are for instance alkyl ethers and cyclic ethers, e.g. methyl isopropyl ether, diethyl ether, dioxane, tetrahydrofuran, etc., alcohols and glycols, e.g. isopropanol, ethylene glycol, ethylene glycol mono-methyl or mono-ethyl-ether, etc., dimethylsulfoxide, and the like solvents. When the reaction described in the first step is over, the inorganic salts are removed by filtration, the solvent is distilled off and the desired arginine di-trityl derivative is recovered according to the conventional methods. As an example, the residue is extracted with a polar aprotic organic solvent, e.g. ethyl ether, the organic layer is washed with water to remove any unreacted arginine or mono-trityl-arginine, and dried. Evaporation of the solvent thus gives the desired di-trityl intermediate.

According to the second step of the above Scheme, this intermediate is selectively de-tritylated on the α-amino nitrogen atom. This step is actually carried out by treating the di-trityl derivative (II) with hydrochloric acid in the presence of a polar aprotic solvent which is capable of dissolving the di-trityl intermediate (II) and let the hydrochloride easily precipitate, recovering the precipitate by filtration and treating it with a protic solvent, e.g. methanol, ethanol, or water. The hydrochloride of formula (III) is then readily precipitated from the obtained solution by the addition of a non-solvent. Typically said non-solvent is an alkyl ether or a cyclic ether.

The obtained product is recovered by conventional methods and carefully washed with suitable solvents. It is then used in the third and last step of the above Scheme wherein the fluorenylmethoxycarbonyl group is introduced into the α-amino nitrogen. To this purpose, the intermediate of formula (III) may be reacted with a reactive derivative of fluorenylmethoxycarbonyl acid of formula (IV) wherein X is a halogen atom, an azido group, or a succinimido group, in the presence of a diluted aqueous solution of an alkaline carbonate.

Preferably, X represents chlorine, as fluorenylmethoxy-carbonyl chloride is the commercial product used for preparing all the other reactive derivatives, and also because the alkaline chloride which is thus obtained can be easily disposed of.

The carbonate solution employed in this step must be sufficiently diluted to bring the pH to a slightly basic value, because, under more basic pH conditions, such as those used for the removal of the base-labile Fmoc group, obviously the reaction does not proceed.

The reaction is carried out at low temperature, generally lower than 10 ° C, and, preferably, comprised between 0 and 4 ° C, by dripping a solution of the reactant of formula (IV) and the carbonate solution into a solution of intermediate (III) in a polar organic solvent optionally mixed with water. The reaction is generally complete in a period of time comprised between 10 and 60 minutes.

Work-up of the mixture containing the desired product of formula (I) according to conventional methods, leads to recovery of the desired compound in high purity. In particular, optimum results are obtained by removing the solvent at room temperature under vacuum, repeatedly triturating the residue with ethyl ether, taking it up in slightly acidic water (pH 3), then extracting the aqueous solution with ethyl acetate and finally recovering the desired product of formula (I) from the organic extracts by evaporating off the solvent.

The thus obtained compound of formula (I) may then be purified by crystallization or chromatography.

An alternative and preferred method for introducing the fluorenylmethoxycarbonyl protection into the arginine α-amino group, consists in treating the hydrochloride of formula (III) with at least a double molar proportion, but preferably an excess, of bis-(trimethylsilyl)acetamide, at a temperature lower than 20-22 ° C, and in the presence of a polar aprotic organic solvent, treating the obtained intermediate $N^{\alpha}$-trimethylsilyl-$N^{G}$-trityl-arginine trimethylsilyl ester with fluorenylmethoxycarbonyl chloride, and finally recovering the desired compound of formula (I) by evaporating off the solution.

The new protected arginine derivative of the present invention may very conveniently be employed in homogeneous solution as well as solid-phase synthesis of peptides containing one or more arginine moieties.

The use of the new derivative of the present invention offers remarkable advantages, particularly in the solid-phase procedure on polyacrylamide supports.

Peptides of actual pharmacological interest which may be prepared by using the new derivative of formula (I) are, for instance, thymopoietin, a thymic hormone with immunomodulating activity, thymopentin, a synthetic pentapeptide corresponding to amino acid residues 32-36 of thymopoietin (i.e.H-Arg-Lys-Asp-Val-Tyr-OH), thymopentin analogs, such as those described in EP-A-0037246 or USP 4,305,852, the human atrial natriuretic peptide (α-hANP) described in EP-A-0147193, analogs thereof, such as for instance those described in WO-A-8504870, and in EP-A-0142487, neurotensin and neurotensin analogs or fragments, etc.. Trial peptide syntheses carried out to evaluate the different coupling methods have shown that the symmetrical anhydride method can suitably be employed only when the desired peptide contains only one arginine residue at the terminal position, while, in general, the cleanest and most efficient method is the DCCI/excess HOBt method.

A third object of the present invention is the use of the novel protected arginine derivative in peptide synthesis and more particularly its use in solid-phase synthesis on polyacrylamide supports, by the activated ester coupling method with in situ production of the activated ester by treatment with DCCI/excess HOBt.

The following examples describing the preparation of the new arginine derivative of formula (I) and its use in peptide synthesis are only aimed at better illustrating representative embodiments of the claimed invention. It has to be understood that these examples are not to be construed as a limitation to the scopes of the invention itself.

Example 1

$N^{\alpha}$-Fluorenylmethoxycarbonyl-$N^{G}$-trityl-arginine

a) Synthesis of $N^{G}$-trityl-arginine hydrochloride

Arginine (8.70 g, 50 mmol), KI (5 g) and a mixture dioxane/1N NaOH 1/1 (120 ml) are poured into a 1000-ml three-necked flask equipped with a mechanical stirrer and two 250-ml dropping funnels, and the

obtained reaction mixture is heated to 45 ° C. One of the dropping funnels is charged with a solution of trityl chloride (41.7 g, 150 mmol) in dioxane (160 ml), while the other is charged with 1N NaOH (160 ml). The two solutions are then simultaneously dripped into the vigorously stirred mixture in about 1/2 hour.

After additional 10 minutes at 45 ° C, the pH is brought to 6 by the addition of acetic acid, and the reaction mixture is extracted with ethyl ether (3 × 300 ml). The organic extracts are combined, washed with water (4 ×200 ml), dried over $Na_2SO_4$ and concentrated to dryness. Anhydrous ethyl ether (1000 ml) is then added to the obtained dry residue under nitrogen atmosphere and tetrahydrofuran is then added thereto until the solid completely dissolves. Anhydrous ethyl ether (100 ml) containing HCl (20 mmol) is added to the vigorously stirred solution kept under nitrogen atmosphere and a yellow solid precipitates. The reaction mixture is stirred at 0 ° C, for 1 hour, then the solid is recovered by filtration, carefully washed with anhydrous ethyl ether, and dried under nitrogen atmosphere. The obtained product is dissolved in methanol (50 ml) and stirred at room temperature for 2 hours. The thick oil which separates upon the addition of ethyl ether (400 ml) is recovered by decantation, washed with ethyl ether and evaporated under vacuum 133,8 Pa (1 mmHg).

$N^G$-trityl-arginine hydrochloride (7.05 g, 31 % yield, calculated on the starting arginine) is thus obtained.

b) Synthesis of $N^\alpha$-fluorenylmethoxycarbonyl-$N^G$-trityl-arginine

Bis-(trimethylsilyl)acetamide (BSA) (10 ml, 41 mmol) is then added to a solution of the above intermediate (6.5 g) in tetrahydrofuran (400 ml) and the obtained reaction mixture is vigorously stirred until a clear solution is obtained (about 40 minutes).

The obtained solution is then cooled to 0 ° C under vigorous stirring and a solution of fluorenylmethoxycarbonyl chloride (2.58 g, 10 mmol) in tetrahydrofuran (50 ml) is dropwise added thereto over 30 minutes.

At the end of the reaction, methanol is added, the solution is concentrated to dryness and the residue is taken up in a small amount of a mixture $CH_2Cl_2$/MeOH 9/1 and applied to a silica gel column (70-230 mesh) prepared in and developed with the same solvent system ($CH_2Cl_2$/MeOH 9/1).

The last eluting fractions, which contain the desired product, are then combined and evaporated to dryness yielding the desired $N^\alpha$-fluorenylmethoxycarbonyl-$N^G$-trityl-arginine (3.9 g), which is finally purified by crystallization from ethyl acetate/ethyl ether.

The thus obtained product showed to be pure both in tlc and hplc (Column : Bondapack® C-18; solvent : linear gradient from 63/37/0.1 to 80/80/0.1 $H_2O/CH_3CN$/TFA in 20 minutes; retention time = 18 minutes).

By treatment of a sample of the above compound with TFA-$H_2O$ 9/1 (v/v), a product is obtained which is co-eluted with an authentic sample of Fmoc-Arg-OH.

M.p. 168-70 ° C

$[\alpha]_D^{25}$ = 7.5 ° (c 0.5, EtOH)

IR absorption maxima ($cm^{-1}$) : $3060_{(=CH\ arom.)}$, $2943_{(C\text{-}H\ aliph.)}$, 1717, 1638, 1597, 1491, $1447_{(C=C\ arom.)}$, 741, $702_{(CH\ mono\text{-}subst.\ arom.)}$.

$^1H$ N.M.R. (DMSO $d_6$) ( $\delta$ ) : 9.8 (s, broad, 1H, COOH); 7.887 (d, 2H, 4-5 Fmoc); 7.669 (t, 2H, 1-8 Fmoc); 7.379 (t, 2H, 3-6 Fmoc); 7.318 (t, 2H, 2-7 Fmoc); 7.28 (m, 15 H, trityl); 6.300 (d, broad, 1H, NH); 4.24-4.19 (m, 3H, 9-10 Fmoc); 3.574 (q, broad, 1H, $\alpha$CH); 3.005 (m, broad, 2H, $\delta CH_2$); 1.505 (m, broad, 2H, $\beta CH_2$); 1.314 (m, broad, 2H, $\gamma CH_2$).

Example 2

$N^\alpha$-Fluorenylmethoxycarbonyl-$N^G$-trityl-arginine

a) Synthesis of $N^G$-trityl-arginine hydrochloride

Five portions of commercial trityl chloride (Fluka AG) (total amount : 11 g) are added in 15 minutes to a mixture of arginine (1.77 g, 10 mmol), KI (10 g), KOH (10 g), 2-methoxyethanol (10 ml) and dioxane (10 ml) heated to 100 ° C. The reaction mixture is allowed to stand at room temperature for additional 30 minutes, the solid residue is removed by filtration, washing it with dioxane, and the filtrate is brought to dryness by evaporating off the solvents at 40 ° C and 1 mmHg. The residue is extracted with ethyl ether (4 × 100 ml). The ether extracts are combined, washed with water a few times to remove unreacted arginine and mono-trityl arginine. The organic phase is dried over $Na_2SO_4$, concentrated to dryness under strictly anhydrous conditions and the residue is taken up with anhydrous ethyl ether (500 ml). A solution of HCl (5 mmol) in anhydrous ethyl ether (10 ml) is vigorously stirred into the above solution.

A precipitate forms which is recovered by filtration under nitrogen atmosphere and carefully washed

with ethyl ether. The washed precipitate is dissolved in methyl alcohol and stirred for 1 hour. The solution is then brought to dryness and the dry residue is washed with ethyl ether (3 × 50 ml). IR and FAB-Mass analyses are carried out on this residue (1.8 g, corresponding to a 40 % yield on the starting arginine) and confirm the structure of the intermediate $N^G$-trityl-argininehydrochloride.

IR absorption maxima ($cm^{-1}$) : $3445_{(NH\ stretching)}$, $3058_{(=C\text{-}H\ arom.\ stretching)}$, $1630_{(C=N\ stretching)}$, 1597, 1492, $1448_{(C=C\ arom.)}$, 750, $704_{(CH\ monosubst.arom.)}$.

In FAB-Mass spectrometry, using glycerol as the matrix, a strong signal is observed corresponding to the protonated molecular ion $MH^+$, m/z 417.

b) Synthesis of $N^\alpha$-fluorenylmethoxycarbonyl-$N^G$-trityl-arginine

The thus obtained intermediate is dissolved in dioxane (15 ml) and water (6 ml), and a solution of fluorenylmethoxycarbonyl chloride (1.03 g) in dioxane (20 ml) and 10% aqueous $Na_2CO_3$ (20 ml) are simultaneously dripped into the thus obtained solution, cooled to 0 ° C.

The reaction mixture is allowed to stand for additional 15 minutes, the solvent is evaporated off under vacuum (1 mmHg) at room temperature, and the residue is triturated with ethyl ether (4 × 20 ml). The residue is then taken up with water/acetic acid (pH 3) and the aqueous mixture is extracted with ethyl acetate (4 × 30 ml). The organic extracts are combined, dried over $Na_2SO_4$ and concentrated to dryness yielding a spongy residue (1.78 g, corresponding to a 70 %).

This raw product is crystallized from ethyl acetate/ethyl ether yielding 1.26 g of pure $N^\alpha$-fluorenylmethoxycarbonyl-$N^G$-trityl-arginine.

The obtained product showed to be pure both in tlc and in hplc (Column : µ Bondapack® C-18; Solvent : linear gradient from 63/37/0.1 to 80/80/0.1 $H_2O/CH_3CN$/TFA in 20 minutes; retention time ≃ 18 minutes). By treatment with TFA-$H_2O$ 9/1 (v/v), a product is obtained which is co-eluted with an authentic sample of Fmoc-Arg-OH.

The obtained product has the same characteristic chemico-physical data as in example 1.

Example 3

Use of the compound of example 1 in the synthesis of thymopentin

Commercial poly(dimethylacrylamide) (1 g) functionalized with a 4-hydroxymethyl-phenoxy-acetyl residue (Pepsyn A 1 meq/g by British C.R.B.) is swollen in dimethylformamide (DMF) and then esterified by treatment with a solution of ($N^\alpha$-Fmoc-($Bu^t$)Tyr)$_2$O (3.6 mmol) in DMF (16 ml), in the presence of N-methylmorpholine (NMM - 3.6 mmol) and 4-dimethylaminopyridine (DMAP - 0.036 mmol), for 30 minutes. The resin thus esterified with the residue $N^\alpha$-Fmoc-($Bu^t$)Tyr (0.55 meq/g), undergoes the standard solid-phase synthetic cycle containing sequential deprotection-washing-coupling steps according to the method described by Sheppard et al. in J.Chem.Soc. Perkin I, 583, (1984), carried out in a Beckman 990B Peptide Synthesizer.

The amino acid sequence Arg-Lys-Asp-Val-Tyr, is assembled one residue at a time and coupling reactions, apart from that for arginine residue, are carried out using preformed symmetrical anhydrides.

The arginine residue is introduced as the active ester of hydroxybenzotriazole, prepared in situ at a concentration which is double than that generally employed in this technique, and using a double molar amount of 1-hydroxybenzotriazole (HOBt) with respect to arginine. Side-chain functionalities, if any, are protected according to a scheme conventional for solid-phase synthesis (tyrosine hydroxy group is protected as the corresponding tert-butyl ether, aspartic acid carboxy group is protected as the corresponding tert-butyl ester, and lysine amino group is protected as the corresponding tert-butoxycarbonyl derivative), with the exception of arginine wherein the guanidino function is protected, according to the method of the present invention, with the trityl group. Following the addition of the last amino acid and its deprotection, the resin is carefully washed with methanol and then with ethyl ether, and finally it is dried for 10 hours at room temperature and 133,8 Pa (1 mmHg), yielding 1.4 g of dried resin.

Part of the foregoing peptide resin (100 mg) is poured into a 50-ml flask and treated with ethandithiol (2 ml), to wet the whole resin, and then with trifluoroacetic acid (18 ml). After 2 hours at room temperature, the reaction mixture is filtered, washing the solid on filter with 10% ethandithiol-trifluoroacetic acid (3 × 2 ml). Water (50 ml) is added to the filtrate and the mixture is then concentrated to dryness at room temperature and 133,8 Pa (1 mmHg). The crude residue which is thus obtained is taken up in water (10 ml) and washed with ethyl ether (2 × 5 ml). The aqueous phase is concentrated to dryness and taken up in water (10 ml).

Acid hydrolysis and amino acid analysis of the thus obtained product gives an 80% yield calculated on

the first amino acid residue incorporated.

Example 4

Use of the compound of example 1 in the synthesis of human Atrial Natriuretic Peptide.

a) Synthesis of the S-acetamidomethyl derivative of human Atrial Natriuretic Peptide.

Commercial poly(dimethylacrylamide) (1 g) functionalized with a 4-hydroxymethyl-phenoxy-acetyl residue (Pepsyn A 1 meq/g by British C.R.B.) is swollen in DMF and esterified by treatment with a solution of ($N^\alpha$-Fmoc-($Bu^t$)Tyr$)_2$O (3.6 mmol) in DMF (16 ml) in the presence of NMM (3.6 mmol) and DMAP (0.036 mmol), for 10 minutes. The resin thus esterified with the residue ($N^\alpha$-Fmoc-($Bu^t$)Tyr (0.2 meq/g), is further esterified with acetic anhydride, in the same conditions for 30 minutes. The standard solid-phase synthetic cycle containing sequential deprotection-washing-coupling steps according to the method described by Sheppard et al. in J.Chem.Soc. Perkin I, 583, (1984), is then carried out in a Beckman 990B Peptide Synthesizer.

The following 24 amino acid residues of the human Atrial Natriuretic Peptide

H-Ser-Leu-Arg-Arg-Ser-Ser-Cys-Phe-Gly-Gly-Arg-Ile-

-Phe-Gln-Asn-Cys-Gly-Leu-Gly-Ser-Gln-Ala-Gly-

-Arg-Tyr-OH

are added sequentially, using, apart from asparagine and glutamine residues, the N-hydroxybenzotriazole active ester procedure, wherein the N-hydroxybenzotriazole ester is prepared in situ by treatment with dicyclohexylcarbodiimide and excess N-hydroxybenzotriazole. Also arginine is introduced as hydroxybenzotriazole ester formed in situ (2 HOBt/DCCl) at a concentration double than that usually employed in this technique (i.e. molar ratios DCCl : HOBt : amino component : Fmoc-Arg(Trt)-OH = 10 : 20 : 1 : 10).

Asparagine and glutamine are introduced using p-nitrophenyl esters in the presence of N-hydroxybenzotriazole.

Side chains are protected as tert-butyl ethers (tyrosine and serine), tert-butyl ester (aspartic acid), and S-acetamidomethyl derivative (cysteine).

Following the addition of the last amino acid and its deprotection, the resin is carefully washed with methanol and then with ethyl ether, and finally it is dried for 10 hours at room temperature and 133,8 Pa (1 mmHg).

Part of the foregoing peptide resin (100 mg) is poured into a 50-ml flask and wetted with ethandithiol (2 ml) first, and then with 5 % (S-acetamidomethyl-cysteine)-trifluoroacetic acid (18 ml).

After 2 hours at room temperature, the mixture is filtered under nitrogen atmosphere washing the solid on filter with 10 % ethandithiol-10 % (S-acetamidomethyl)-cysteine-trifluoroacetic acid (3 $\times$ 2 ml). Water (50 ml) is added to the filtrate and the mixture is then concentrated to dryness at room temperature and 133,8 Pa (1 mmHg). The crude residue which is thus obtained is taken up in water (10 ml) and washed with ethyl ether (2 $\times$ 5 ml). The aqueous phase is again concentrated to dryness and taken up in 50 mM ammonium acetate buffer pH 4.5 (1 ml).

The obtained solution is applied to a column of Sephadex C-25®, prepared in the same buffer, and eluted with a linear gradient from 50 mM to 0.5 M ammonium acetate buffer (pH 7), over 4 hours.

The last eluting peak is collected and lyophilized three times in order to remove ammonium acetate.

Acid hydrolysis of the obtained product with 6N HCl, and amino-acid analysis gave the following results :

Ala 1.00; Asx 2.10; Ser 5.00; Glx 1.10; Gly 5.09; Ile 0.98; Leu 2.00; Tyr 1.01; Phe 2.08; Arg 4.95;

The overall yield, calculated on the first amino acid residue which has been incorporated, is 75 %. Analytical reversed-phase hplc of crude $\alpha$-hANP(S-Acm$)_2$(Column : Lichrosorb® RP-18, 10$\mu$m, 4 $\times$ 250 mm; Solvent : linear gradient from 10 % to 50 % $CH_3CN$ in 0.1 % TFA over 50 minutes; Flow rate : 1.5 ml/min) gave a single peak.

If desired this product can be further purified by reversed-phase chromatography using a Lichroprep® RP-18 column and eluting with a mixture $H_2O/CH_3CN$/TFA 82%/18%/0.2% for the first 35 minutes, and then with a mixture of the same solvents in the following % : 73/27/0.2. Fractions are collected with a flow rate of

7ml/min. Fractions containing the purified peptide are combined, concentrated to dryness, lyophilized and taken up in water; amino-acid analysis gives 5.67 µmol of peptide corresponding to a chromatographic yield of 70 %.

b) Synthesis of human Atrial Natriuretic Peptide

2 µmol of the above compound, lyophilized, are taken up in acetic acid/water (90/10) (0.65 ml) containing HCl (3 µmol); a 50 mM solution of $I_2$ in acetic acid (2.5 ml) is then added thereto and the reaction is allowed to proceed for 16 minutes. Powdered zinc (0.5 g) is then added to the reaction mixture and, 5 minutes later, water (10 ml) is also added. The resulting mixture is then filtered and the filtrate is concentrated to dryness. The obtained residue is purified by reversed-phase hplc as described under item a) above.

By evaporating off and lyophilizing the fractions containing the pure peptide, the compound of the title (1.4 µmol, corresponding to 70 % yield, calculated on the starting S-acetamidomethyl derivative) is obtained. Amino acid analysis of this product, following acid hydrolysis with 6N HCl, is as follows :
Ala 1; Asp 1.94; Ser 4.7; Gln 1.07; Gly 5.01; Ile 0.90; Leu 2.09; Tyr 0.95; Phe 2; Arg 4.95; Met 0.92; Cys 0.95
The disulfide bridge in the product structure has been confirmed by i) comparing the amount of cysteic acid obtained upon oxidation with performic acid with the theoretical value (see Moore G. in J.Biol.Chem. 238, 255 (1963)) and ii) simultaneously confirming the absence of free -SH groups by the Ellman method (Habeeb A.F.S. in Methods in Enzymol. 25, 457, (1972)).

**Claims**

**Claims for the following Contracting States : AT, BE, CH, DE, FR, GB, IT, LI, LU, NL, SE**

**1.** A compound of formula (I)

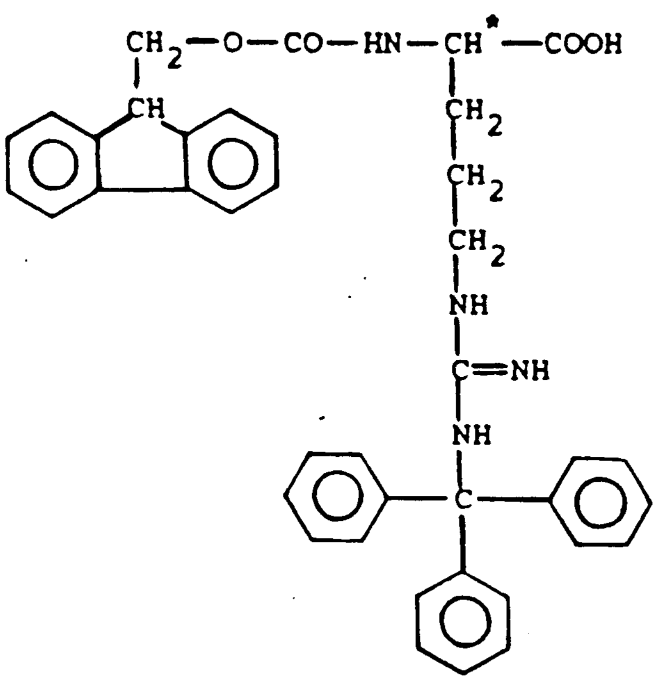

(I)

wherein the asterisk means that the starred carbon atom may have either the R or the S configuration, or the compound may consist of a mixture of the two isomeric forms in any proportion.

**2.** A process for preparing a compound of formula (I)

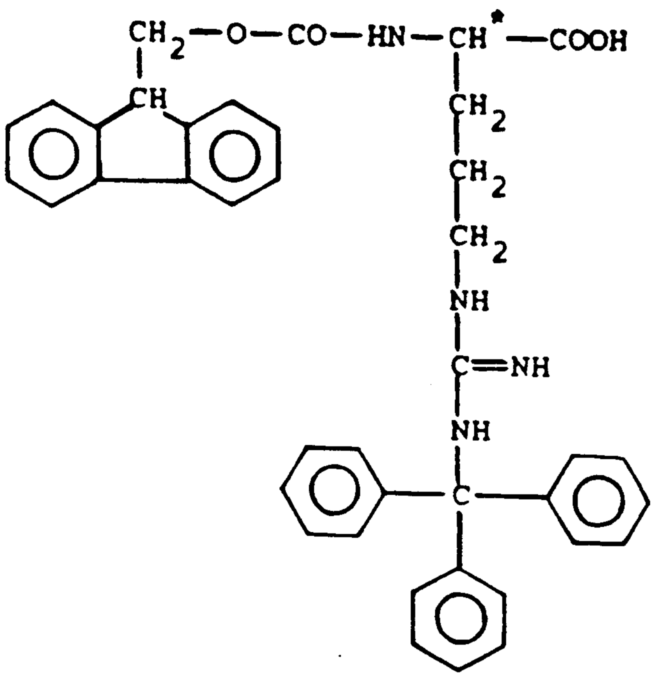

(I)

wherein the asterisk means that the starred carbon atom may have either the R or the S configuration, or the compound may consist of a mixture of the two isomeric forms in any proportion,
which comprises

a) forming the $N^{\alpha}$-trityl-$N^{G}$-trityl-arginine of formula (II)

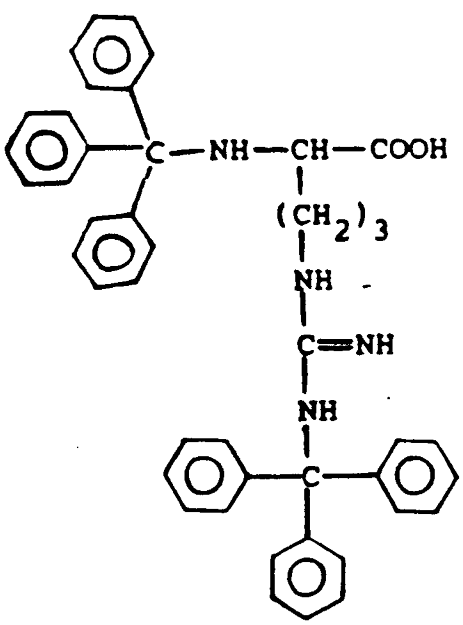

(II)

by reacting L-arginine or D-arginine or a mixture of the two isomeric forms in any proportion, with at least a double molar amount of trityl iodide, in the presence of an inorganic base which acts as the acceptor of the hydriodic acid which forms,

b) selectively cleaving the trityl group on the $\alpha$-$NH_2$ group by treatment of the di-trityl derivative of formula (II) with hydrochloric acid in the presence of a protic solvent, and

c) introducing the fluorenylmethoxycarbonyl group on the $\alpha$-$NH_2$ by reacting the intermediate

hydrochloride of formula (III)

(III)

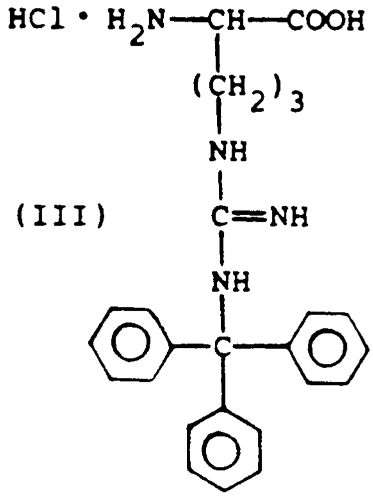

either with a reactive derivative of fluorenylmethoxycarbonyl acid of formula (IV)

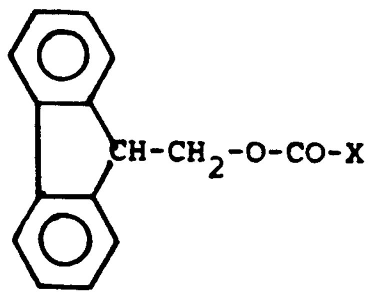

(IV)

wherein X represents a chlorine atom, an azido group or a succinimido group, in the presence of a diluted aqueous solution of an alkaline carbonate, or with at least a double molar amount of bis(trimethylsilyl)acetamide and replacing the $\alpha$-$NH_2$ trimethylsilyl group with fluorenylmethoxycarbonyl by treatment with fluorenylmethoxycarbonyl chloride.

**3.** A process as in claim 2 wherein step a) is carried out with 50 to 100 % molar excess trityl iodide with respect to the stoichiometric amount needed.

**4.** A process as in claim 2 wherein trityl iodide is formed in situ by the addition of trityl chloride to a mixture of arginine, inorganic base and alkaline iodide.

**5.** A process as in claim 2 wherein the inorganic base is an alkaline or alkaline-earth metal hydroxide.

**6.** A process as in claim 2 wherein step b) is carried out in the presence of a polar aprotic organic solvent which is capable of dissolving the di-trityl intermediate (II) and let the hydrochloride which forms easily precipitate.

**7.** A process as in claim 6 wherein said solvent is an alkyl ether or a cyclic ether.

**8.** A process as in claim 2 wherein in step c) X represents a chlorine atom.

**9.** A process as in claim 2 wherein step c) is carried out at a temperature lower than 10 °C.

**10.** A process for preparing an arginine-containing peptide, by stepwise assembly or fragment condensation, either in free solution or by solid-phase, characterized in that the arginine residues are introduced

as the corresponding active esters of $N^{\alpha}$-fluorenylmethoxycarbonyl-$N^{G}$-trityl-arginine.

**11.** A process as in claim 10 wherein the arginine residues are introduced as the corresponding $N^{\alpha}$-fluorenylmethoxycarbonyl-$N^{G}$-trityl-arginine hydroxybenzotriazole ester.

**12.** $N^{\alpha}$-fluorenylmethoxycarbonyl-$N^{G}$-trityl-arginine hydroxybenzotriazole ester.

**13.** A compound of claim 12 obtainable by reacting $N^{\alpha}$-fluorenylmethoxycarbonyl-$N^{G}$-trityl-arginine with N-hydroxybenzotriazole in the presence of dicyclohexylcarbodiimide.

**Claims for the following Contracting States : ES, GR**

**1.** A process for preparing a compound of formula (I)

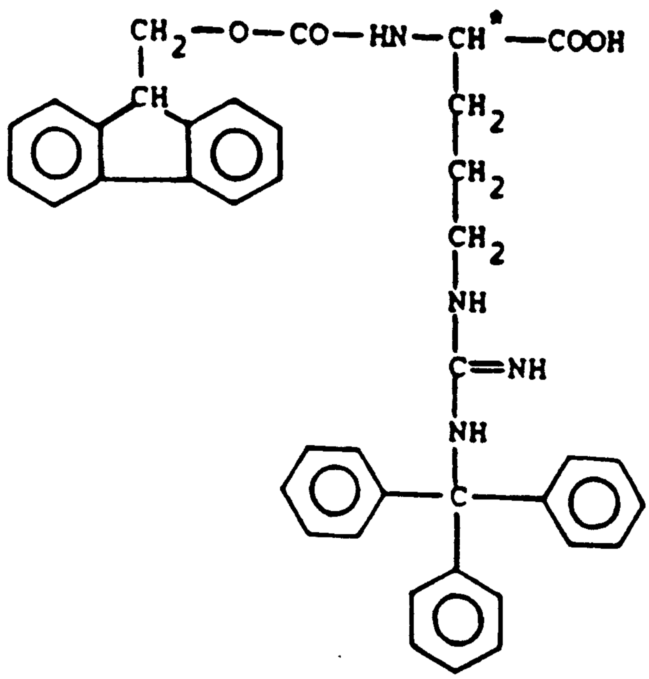

(I)

wherein the asterisk means that the starred carbon atom may have either the R or the S configuration, or the compound may consist of a mixture of the two isomeric forms in any proportion, which comprises

a) forming the $N^{\alpha}$-trityl-$N^{G}$-trityl-arginine of formula (II)

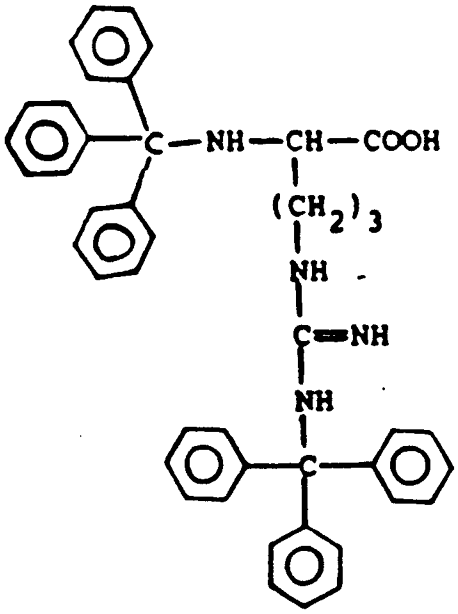

(II)

by reacting L-arginine or D-arginine or a mixture of the two isomeric forms in any proportion, with at least a double molar amount of trityl iodide, in the presence of an inorganic base which acts as the acceptor of the hydriodic acid which forms,

b) selectively cleaving the trityl group on the α-$NH_2$ group by treatment of the di-trityl derivative of formula (II) with hydrochloric acid in the presence of a protic solvent, and

c) introducing the fluorenylmethoxycarbonyl group on the α-$NH_2$ by reacting the intermediate hydrochloride of formula (III)

(III)

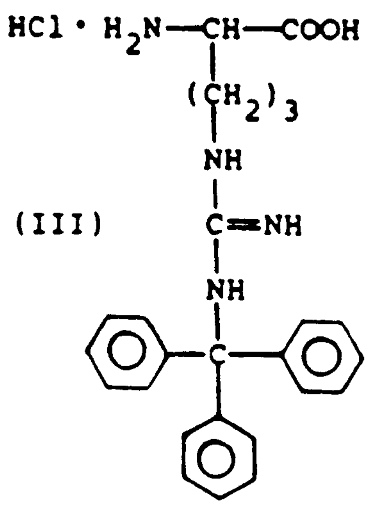

either with a reactive derivative of fluorenylmethoxycarbonyl acid of formula (IV)

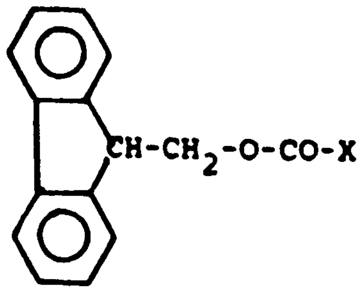

(IV)

wherein X represents a chlorine atom, an azido group or a succinimido group, in the presence of a diluted aqueous solution of an alkaline carbonate, or with at least a double molar amount of bis(trimethylsilyl)acetamide and replacing the $\alpha$-$NH_2$ trimethylsilyl group with fluorenylmethoxycarbonyl by treatment with fluorenylmethoxycarbonyl chloride.

**2.** A process as in claim 1,wherein step a) is carried out with 50 to 100 % molar excess trityl iodide with respect to the stoichiometric amount needed.

**3.** A process as in claim 1, wherein trityl iodide is formed in situ by the addition of trityl chloride to a mixture of arginine, inorganic base and alkaline iodide.

**4.** A process as in claim 1, wherein the inorganic base is an alkaline or alkaline-earth metal hydroxide.

**5.** A process as in claim 1, wherein step b) is carried out in the presence of a polar aprotic organic solvent which is capable of dissolving the di-trityl intermediate (II) and let the hydrochloride which forms easily precipitate.

**6.** A process as in claims 5, wherein said solvent is an alkyl ether or a cyclic ether.

**7.** A process as in claim 1, wherein in step c) X represents a chlorine atom.

**8.** A process as in claim 2, wherein step c) is carried out at a temperature lower than 10 ° C.

**9.** A process for preparing an arginine-containing peptide, by stepwise assembly or fragment condensation, either in free solution or by solid-phase, characterized in that the arginine residues are introduced as the corresponding active esters of $N^{\alpha}$-fluorenylmethoxycarbonyl-$N^{G}$-trityl-arginine.

**10.** A process as in claim 9, wherein the arginine residues are introduced as the corresponding $N^{\alpha}$-fluorenylmethoxycarbonyl-$N^{G}$-trityl-arginine hydroxybenzotriazole ester.

**Revendications**
**Revendications pour les Etats contractants suivants : AT, BE, CH, DE, FR, GB, IT, LI, LU, NL, SE**

**1.** Composé de formule (I)

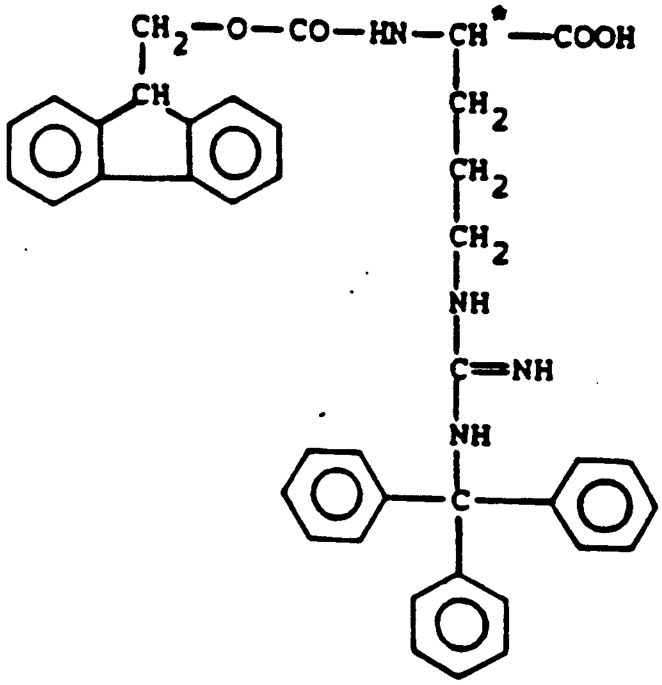

(I)

dans laquelle l'astérisque signifie que l'atome de carbone ainsi marqué peut avoir soit la configuration R soit la configuration S, ou que le composé peut consister en un mélange des deux formes isomères dans des proportions quelconques.

**2.** procédé de préparation du composé de formule (I)

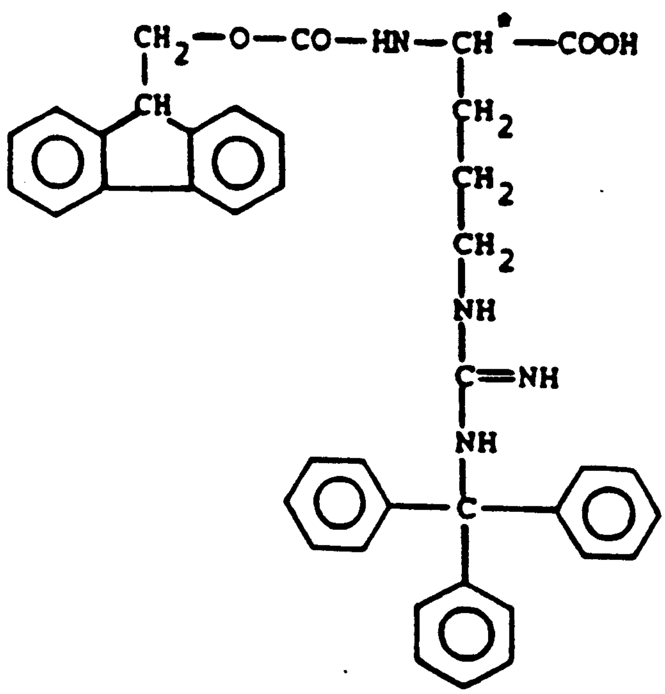

(I)

dans laquelle l'astérisque Signifie que l'atome de carbone ainsi marqué peut avoir soit la configuration R soit la configuration S, ou que le composé peut consister en un mélange des deux formes isomères dans des proportions quelconques, qui comprend les étapes suivantes :

a) formation de la $N^{\alpha}$-trityl-$N^{G}$-trityl-arginine, de formule (II)

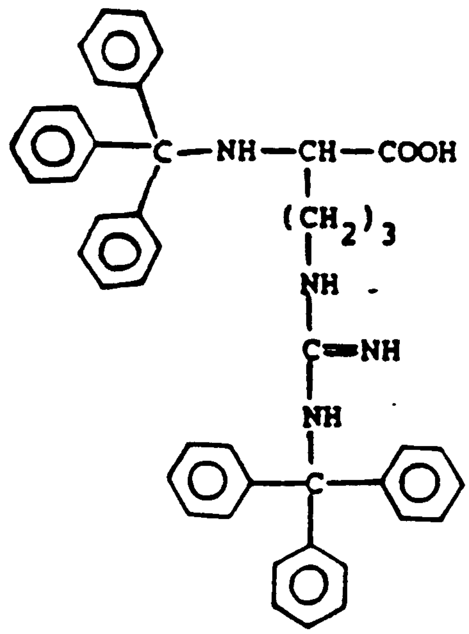

(II)

en faisant réagir la L-arginine ou la D-arginine ou un mélange des deux formes isomères dans des proportions quelconques, avec une quantité au moins bimolaire d'iodure de trityle, en présence d'une base minérale qui intervient en tant qu'accepteur de l'acide iodhydrique qui se forme,

b) coupure sélective du groupe trityle sur le radical $\alpha$-$NH_2$,par traitement du dérivé di-trityle de formule (II) avec l'acide chlorhydrique en présence d'un solvant protique, et

c) introduction du groupe fluorénylméthoxycarbonyle sur le radical $\alpha$-$NH_2$, en faisant réagir l'intermédiaire chlorhydrique de formule (III)

(III)

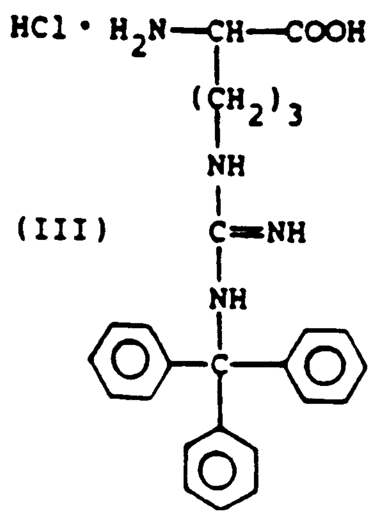

soit avec un réactif dérivé de l'acide fluorénylméthoxycarbonyle de formule (IV)

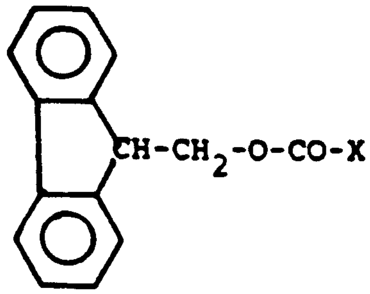

(IV)

dans laquelle X représente un atome de chlore, un groupe azido ou un groupe succinimido, en présence d'une solution aqueuse d'un carbonate alcalin, soit avec une quantité au moins bimolaire de bis (triméthylsilyl) acétamide et en remplaçant le groupe $\alpha$-$NH_2$ triméthylsilyl par le fluorénylméthoxycarbonyle en traitant avec le chlorure de fluorénylméthoxycarbonyle.

**3.** Procédé tel que revendiqué dans la revendication 2, dans lequel l'étape a) est effectuée avec de l'iodure de trityle en excès molaire de 50 à 100 % par rapport à la quantité stoechiométrique nécessaire.

**4.** Procédé tel que revendiqué dans la revendication 2, dans lequel l'iodure de trityle est formé in situ par addition du chlorure de trityle à un mélange d'arginine, d'une base minérale et d'un iodure alcalin.

**5.** Procédé tel que revendiqué dans la revendication 2, dans lequel la base minérale est un hydroxyde de métal alcalin ou alcalino-terreux.

**6.** Procédé tel que revendiqué dans la revendication 2, dans lequel l'étape b) est effectuée en présence d'un solvant organique polaire aprotique qui est capable de dissoudre l'intermédiaire di-trityle (II) et de laisser l'hydrochlorure qui forme facilement un précipité.

**7.** Procédé tel que revendiqué dans la revendication 6, dans lequel le solvant est un éther d'alcoyle ou un éther cyclique.

**8.** Procédé tel que revendiqué dans la revendication 2, dans lequel dans l'étape c) le terme X représente un atome de chlore.

**9.** Procédé tel que revendiqué dans la revendication 2, dans lequel l'étape c) est effectuée à une température inférieure à 10° C.

**10.** Procédé de préparation d'un peptide contenant de l'arginine, par assemblage par degrés successifs ou par condensation de fragments, soit en solution libre soit en phase solide, caractérisé par le fait que les restes d'arginine sont introduits en tant qu'esters actifs correspondants de la $N^{\alpha}$-fluorénylméthoxycarbonyl-$N^{G}$-trityl-arginine.

**11.** Procédé tel que revendiqué dans la revendication 10, dans lequel les restes d'arginine sont introduits en tant qu'ester hydroxybenzotriazole correspondant de la $N^{\alpha}$-fluorénylméthoxycarbonyl-$N^{G}$-trityl-arginine.

**12.** Ester hydroxybenzotriazole de la $N^{\alpha}$-fluorénylméthoxycarbonyl-$N^{G}$-trityl-arginine.

**13.** Composé tel que revendiqué dans la revendication 12, que l'on peut obtenir en faisant réagir la $N^{\alpha}$-fluorénylméthoxycarbonyl-$N^{G}$-trityl-arginine avec le N-hydroxybenzotriazole en présence de dicyclohexylcarbodiimide.

**Revendications pour les Etats contractants suivants : ES, GR**

**1.** procédé de préparation du composé de formule (I)

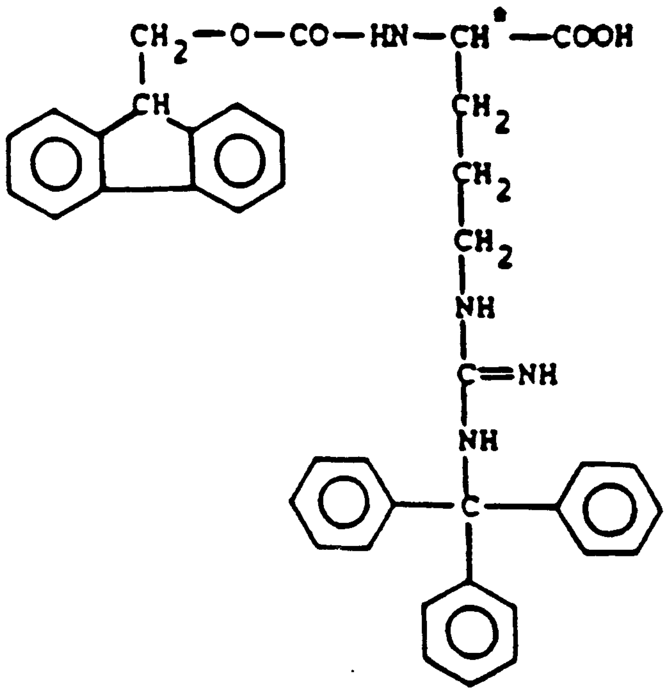

(I)

dans laquelle l'astérisque signifie que l'atome de carbone ainsi marqué peut avoir soit la configuration R soit la configuration S, ou que le composé peut consister en un mélange des deux formes isomères dans des proportions quelconques, qui comprend les étapes suivantes :

a) formation de la $N^{\alpha}$-trityl-$N^{G}$-trityl-arginine, de formule (II)

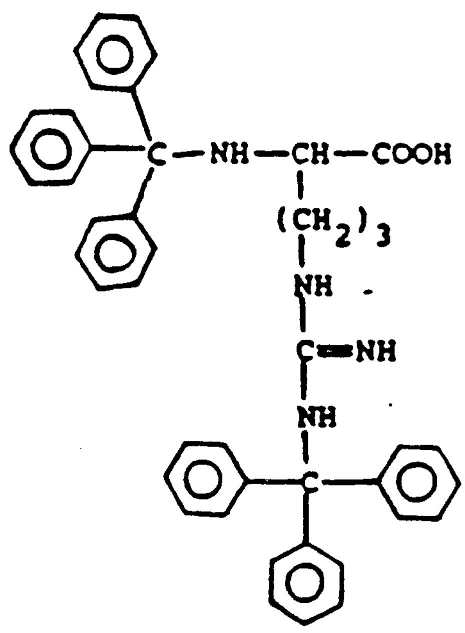

(II)

en faisant réagir la L-arginine ou la D-arginine ou un mélange des deux formes isomères dans des proportions quelconques, avec une quantité au moins bimolaire d'iodure de trityle, en présence d'une base minérale qui intervient en tant qu'accepteur de l'acide iodhydrique qui se forme,

b) coupure sélective du groupe trityle sur le radical $\alpha$-$NH_2$,par traitement du dérivé di-trityle de formule (II) avec l'acide chlorhydrique en présence d'un solvant protique, et

c) introduction du groupe fluorénylméthoxycarbonyle sur le radical $\alpha$-$NH_2$, en faisant réagir l'intermédiaire chlorhydrique de formule (III)

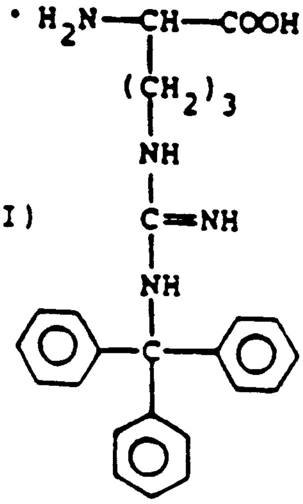

(III)

soit avec un réactif dérivé de l'acide fluorénylméthoxycarbonyle de formule (IV)

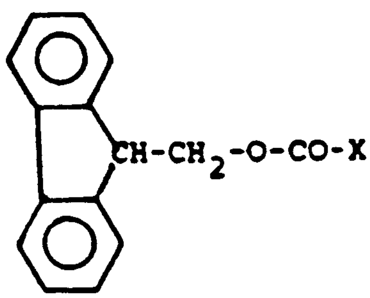

(IV)

dans laquelle X représente un atome de chlore, un groupe azido ou un groupe succinimido, en présence d'une solution aqueuse d'un carbonate alcalin, soit avec une quantité au moins bimolaire de bis (triméthylsilyl) acétamide et en remplaçant le groupe $\alpha$-$NH_2$ triméthylsilyl par le fluorénylméthoxycarbonyle en traitant avec le chlorure de fluorénylméthoxycarbonyle.

**2.** Procédé tel que revendiqué dans la revendication 1, dans lequel l'étape a) est effectuée avec de l'iodure de trityle en excès molaire de 50 à 100 % par rapport à la quantité stoechiométrique nécessaire.

**3.** Procédé tel que revendiqué dans la revendication 1, dans lequel l'iodure de trityle est formé in situ par addition du chlorure de trityle à un mélange d'arginine, d'une base minérale et d'un iodure alcalin.

**4.** Procédé tel que revendiqué dans la revendication 1, dans lequel la base minérale est un hydroxyde de métal alcalin ou alcalino-terreux.

**5.** Procédé tel que revendiqué dans la revendication 1, dans lequel l'étape b) est effectuée en présence d'un solvant organique polaire aprotique qui est capable de dissoudre l'intermédiaire di-trityle (II) et de laisser l'hydrochlorure qui forme facilement un précipité.

**6.** Procédé tel que revendiqué dans la revendication 5, dans lequel le solvant est un éther d'alcoyle ou un éther cyclique.

**7.** Procédé tel que revendiqué dans la revendication 1, dans lequel dans l'étape c) le terme X représente un atome de chlore.

**8.** Procédé tel que revendiqué dans la revendication 1, dans lequel l'étape c) est effectuée à une température inférieure à 10° C.

**9.** Procédé de préparation d'un peptide contenant de l'arginine, par assemblage par degrés successifs ou par condensation de fragments, soit en solution libre soit en phase solide, caractérisé par le fait que les restes d'arginine sont introduits en tant qu'esters actifs correspondants de la $N^{\alpha}$-fluorénylméthoxycarbonyl-$N^{G}$-trityl-arginine.

**10.** Procédé tel que revendiqué dans la revendication 9, dans lequel les restes d'arginine sont introduits en tant qu'ester hydroxybenzotriazole correspodant de la $N^{\alpha}$-fluorénylméthoxycarbonyl-$N^{G}$-trityl-arginine.

**Patentansprüche**

**Patentansprüche für folgende Vertragsstaaten : AT, BE, CH, DE, FR, GB, IT, LI, LU, NL, SE**

**1.** Verbindung der Formel (I)

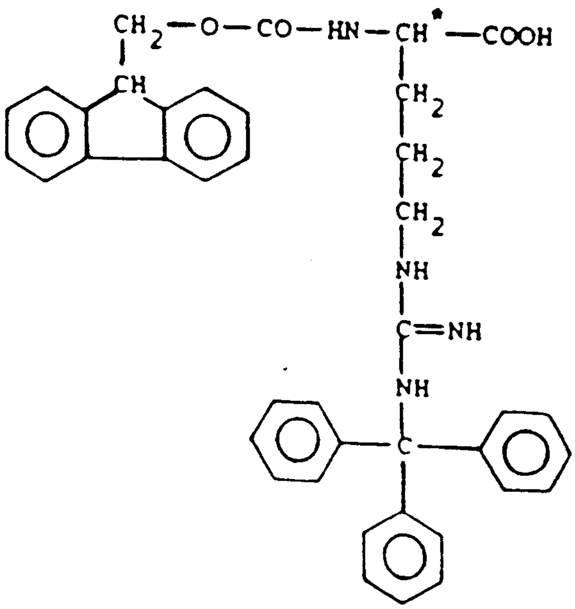

(I),

worin das Sternchen bedeutet, daß das mit dem Sternchen bezeichnete Kohlenstoffatom entweder die R- oder die S-Konfiguration aufweisen kann, oder die Verbindung kann aus einer Mischung der beiden isomeren Formen in jedem Verhältnis bestehen.

**2.** Verfahren zum Herstellen einer Verbindung der Formel (I)

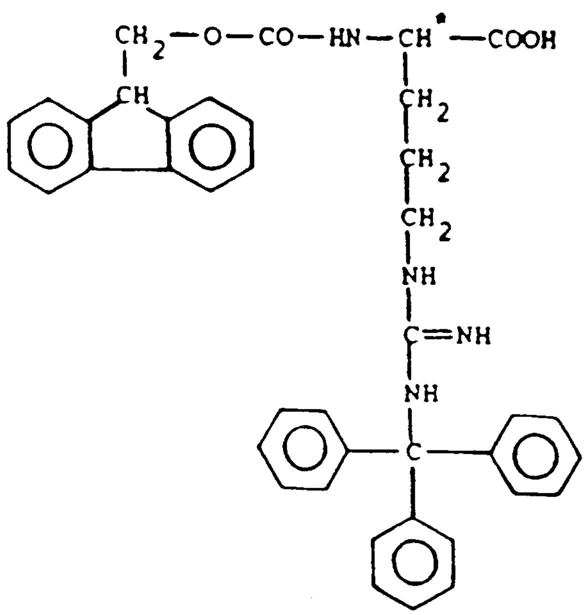

(I),

worin das Sternchen bedeutet, daß das mit dem Sternchen bezeichnete Kohlenstoffatom entweder die R- oder die S-Konfiguration aufweisen kann, oder die Verbindung kann aus einer Mischung der beiden isomeren Formen in jedem Verhältnis bestehen, das

a) das Bilden des $N^{\alpha}$-Trityl-$N^{G}$-tritylarginins der Formel (II)

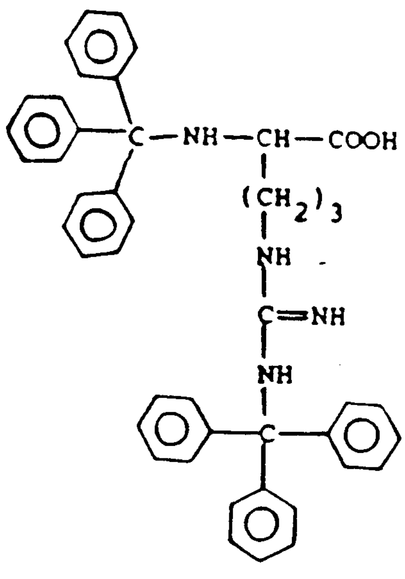

(II)

durch Umsetzen von L-Arginin oder D-Arginin oder einer Mischung der beiden isomeren Formen in jedem Verhältnis mit mindestens einer doppelten molaren Menge Trityliodid in Anwesenheit einer anorganischen Base, die als Akzeptor der sich bildenden Iodwasserstoffsäure wirkt,

b) das selektive Abspalten der Tritylgruppe an der $\alpha$-$NH_2$-Gruppe durch Behandlung des Ditritylderivates der Formel (II) mit Salzsäure in Anwesenheit eines protischen Lösungsmittels und

c) das Einführen der Fluorenylmethoxycarbonylgruppe an der $\alpha$-$NH_2$-Gruppe durch Umsetzen des Hydrochloridzwischenproduktes der Formel (III)

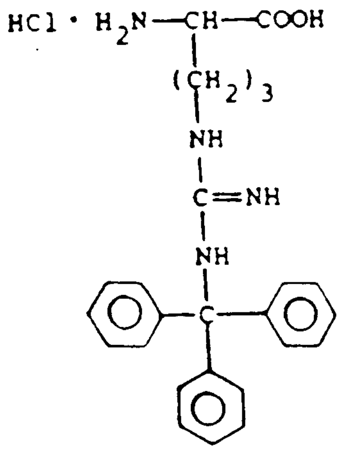

(III)

entweder mit einem reaktiven Derivat der Fluorenylmethoxycarbonylsäure der Formel (IV)

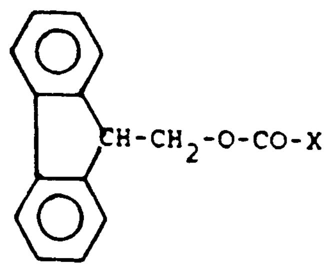

(IV),

worin X ein Chloratom, eine Azidogruppe oder eine Succinimidogruppe bedeutet, in Anwesenheit einer verdünnten wässerigen Lösung eines Alkalicarbonats oder mit mindestens einer doppelten molaren Menge von Bis-(trimethylsilyl)-acetamid und das Ersetzen der $\alpha$-$NH_2$-Trimethylsilylgruppe durch Fluorenylmethoxycarbonyl durch Behandlung mit Fluorenylmethoxycarbonylchlorid umfaßt.

3. Verfahren nach Anspruch 2, in dem Schritt a) mit einem 50 bis 100 % molaren Überschuß von Trityliodid in bezug auf die benötigte stöchiometrische Menge durchgeführt wird.

4. Verfahren nach Anspruch 2, in dem Trityliodid durch Zusatz von Tritylchlorid zu einer Mischung von Arginin, einer anorganischen Base und alkalischem Iodid in situ gebildet wird.

5. Verfahren nach Anspruch 2, in dem die anorganische Base ein Alkali- oder Erdalkalimetallhydroxid ist.

6. Verfahren nach Anspruch 2, in dem Schritt b) in Anwesenheit eines polaren aprotischen organischen Lösungsmittels, das imstande ist, das Ditritylzwischenprodukt (II) zu lösen und das sich bildende Hydrochlorid leicht ausfallen zu lassen, durchgeführt wird.

7. Verfahren nach Anspruch 6, in dem das Lösungsmittel ein Alkyläther oder ein cyclischer Äther ist.

8. Verfahren nach Anspruch 2, in dem in Schritt c) X ein Chloratom bedeutet.

9. Verfahren nach Anspruch 2, in dem Schritt c) bei einer Temperatur von weniger als 10 °C durchgeführt wird.

10. Verfahren zum Herstellen eines argininhaltigen Peptids durch schrittweise Aufbau- oder Fragmentkondensation entweder in freier Lösung oder durch Festphase, dadurch gekennzeichnet, daS die Argininreste als die entsprechenden aktiven Ester von $N^{\alpha}$-Fluorenylmethoxycarbonyl-$N^{G}$-tritylarginin eingeführt

werden.

**11.** Verfahren nach Anspruch 10, in dem die Argininreste als der entsprechende $N^{\alpha}$-Fluo renylmethoxycarbonyl-$N^{G}$-trityl argininhydroxybenzotriazolester eingeführt werden.

**12.** $N^{\alpha}$-Fluorenylmethoxycarbonyl-$N^{G}$-tritylargininhydroxybenzotriazolester.

**13.** Verbindung nach Anspruch 12, erhältlich durch Umsetzen von $N^{\alpha}$-Fluorenylmethoxycarbonyl-$N^{G}$-tritylarginin mit N-Hydroxybenzotriazol in Anwesenheit von Dicyclohexylcarbodiimid.

**Patentansprüche für folgende Vertragsstaaten : ES, GR**

**1.** Verfahren zum Herstellen einer Verbindung der Formel (I)

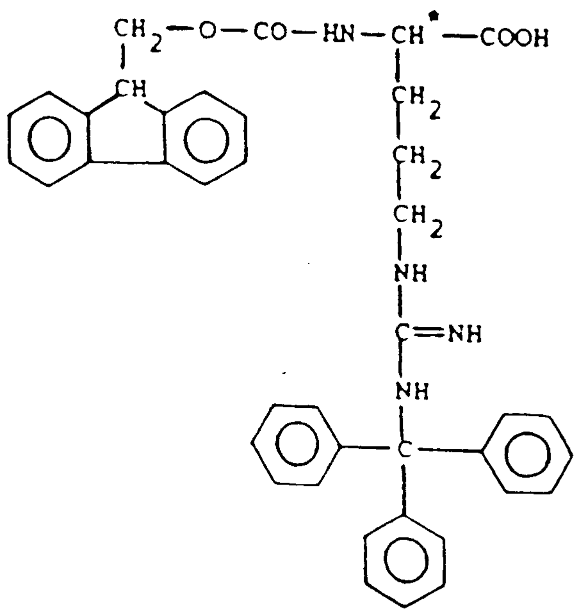

(I),

worin das Sternchen bedeutet, daß das mit dem Sternchen bezeichnete Kohlenstoffatom entweder die R- oder die S-Konfiguration aufweisen kann, oder die Verbindung kann aus einer Mischung der beiden isomeren Formen in jedem Verhältnis bestehen, das

a) das Bilden des $N^{\alpha}$-Trityl-$N^{G}$-tritylarginins der Formel (II)

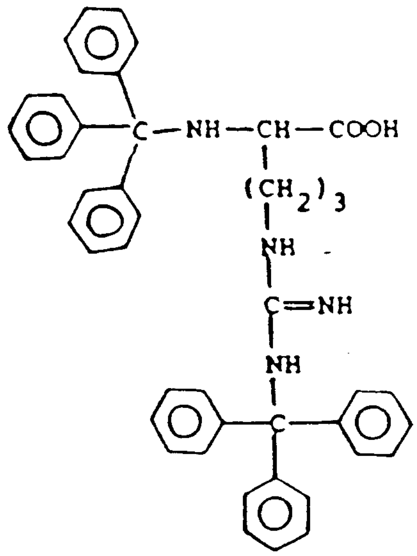

(II)

durch Umsetzen von L-Arginin oder D-Arginin oder einer Mischung der beiden isomeren Formen in jedem Verhältnis mit mindestens einer doppelten molaren Menge Trityliodid in Anwesenheit einer anorganischen Base, die als Akzeptor der sich bildenden Iodwasserstoffsäure wirkt,

b) das selektive Abspalten der Tritylgruppe an der α-$NH_2$-Gruppe durch Behandlung des Ditritylderivates der Formel (II) mit Salzsäure in Anwesenheit eines protischen Lösungsmittels und

c) das Einführen der Fluorenylmethoxycarbonylgruppe an der α-$NH_2$-Gruppe durch Umsetzen des Hydrochloridzwischenproduktes der Formel (III)

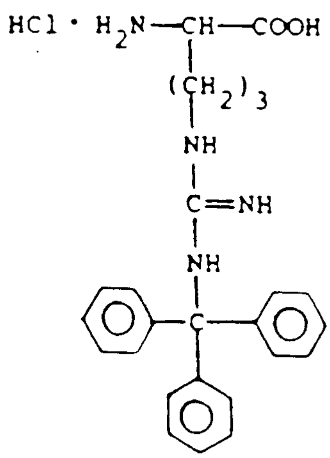

(III)

entweder mit einem reaktiven Derivat der Fluorenylmethoxycarbonylsäure der Formel (IV)

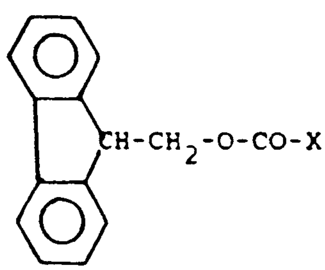

(IV),

worin X ein Chloratom, eine Azidogruppe oder eine Succinimidogruppe bedeutet, in Anwesenheit einer verdünnten wässerigen Lösung eines Alkalicarbonats oder mit mindestens einer doppelten molaren Menge von Bis-(trimethylsilyl)-acetamid und das Ersetzen der $\alpha$-$NH_2$-Trimethylsilylgruppe durch Fluorenylmethoxycarbonyl durch Behandlung mit Fluorenylmethoxycarbonylchlorid umfaßt.

**2.** Verfahren nach Anspruch 1, in dem Schritt a) mit einem 50 bis 100 % molaren Überschuß von Trityliodid in bezug auf die benötigte stöchiometrische Menge durchgeführt wird.

**3.** Verfahren nach Anspruch 1, in dem Trityliodid durch Zusatz von Tritylchlorid zu einer Mischung von Arginin, einer anorganischen Base und alkalischem Iodid in situ gebildet wird.

**4.** Verfahren nach Anspruch 1, in dem die anorganische Base ein Alkali- oder Erdalkalimetallhydroxid ist.

**5.** Verfahren nach Anspruch 1, in dem Schritt b) in Anwesenheit eines polaren aprotischen organischen Lösungsmittels, das imstande ist, das Ditritylzwischenprodukt (II) zu lösen und das sich bildende Hydrochlorid leicht ausfallen zu lassen, durchgeführt wird.

**6.** Verfahren nach Anspruch 5, in dem das Lösungsmittel ein Alkyläther oder ein cyclischer Äther ist.

**7.** Verfahren nach Anspruch 1, in dem in Schritt c) X ein Chloratom bedeutet.

**8.** Verfahren nach Anspruch 1, in dem Schritt c) bei einer Temperatur von weniger als 10 °C durchgeführt wird.

**9.** Verfahren zum Herstellen eines argininhaltigen Peptids durch schrittweise Aufbau- oder Fragmentkondensation entweder in freier Lösung oder durch Festphase, dadurch gekennzeichnet, daß die Argininreste als die entsprechenden aktiven Ester von $N^{\alpha}$-Fluorenymethoxycarbonyl-$N^{G}$-tritylarginin eingeführt werden.

**10.** Verfahren nach Anspruch 9, in dem die Argininreste als der entsprechende $N^{\alpha}$-Fluorenylmethoxycarbonyl-$N^{G}$-tritylargininhydroxybenzotriazolester eingeführt werden.